Europäisches Patentamt

European Patent Office

Office européen des brevets

(11) Numéro de publication: **0 270 400 B1**

# FASCICULE DE BREVET EUROPEEN

(45) Date de publication de fascicule du brevet: **08.07.92** (51) Int. Cl.⁵: **A61F 5/448**

(21) Numéro de dépôt: **87402406.0**

(22) Date de dépôt: **26.10.87**

(54) **Dispositif perfectionné d'ostomie.**

(30) Priorité: **31.10.86 FR 8615187**

(43) Date de publication de la demande:
**08.06.88 Bulletin 88/23**

(45) Mention de la délivrance du brevet:
**08.07.92 Bulletin 92/28**

(84) Etats contractants désignés:
**AT BE CH DE ES GB IT LI NL SE**

(56) Documents cités:
EP-A- 0 149 391   EP-A- 0 163 979
EP-A- 0 171 255   EP-A- 0 197 672
EP-A- 0 227 317   FR-A- 2 387 643
GB-A- 1 021 145   US-A- 3 736 934

(73) Titulaire: **LABORATOIRES BIOTROL**
**1, rue du Foin**
**F-75140 Paris Cedex 03(FR)**

(72) Inventeur: **Holtermann, Henri**
**12, Avenue Pierre Loti**
**F-64500 Saint-Jean-de-Luz(FR)**
Inventeur: **Hamelin, Claude**
**1, Lotissement Aquerrondo Chemin de Fer-**
**res**
**F-64310 Ascain(FR)**

(74) Mandataire: **Orès, Bernard et al**
**Cabinet ORES 6, Avenue de Messine**
**F-75008 Paris(FR)**

**Description**

L'invention a pour objet un dispositif perfectionné d'ostomie.

On sait que certaines interventions chirurgicales du tractus gastro-intestinal ou de l'appareil urinaire s'accompagnent chez le patient ayant subi ces interventions par la création d'un orifice physiologique artificiel, auquel est parfois associé un moyen de recueil de fluides corporels, comme une sonde de recueil d'urine ou analogue. Les fluides évacués ou, dans le cas d'ostomie abdominale, les déchets corporels sont généralement recueillis dans une poche collectrice dont on connaît de nombreuses réalisations.

Dans certaines d'entre elles, prévues pour des dispositifs d'ostomie dits à deux éléments et décrites par exemple, par EP-A-0 171 255 ou par FR-A-2 387 643, la poche collectrice est un sac jetable, ou qui peut être vidé, propre à être rapporté de manière amovible sur un dispositif de bague fixé au corps de l'utilisateur par un patin adhésif et/ou par une ceinture. Dans ces réalisations, de même que dans celle que décrit EP-A-0 197 672 qui vise plus particulièrement un dispositif d'urostomie, la poche collectrice est mise en place, sur une bague ou rebord de montage fixé au corps de l'utilisateur, par un encliquetage résultant d'une poussée ou pression qu'applique celui-ci autour de l'ouverture d'ostomie pour faire coopérer à emboîtement étanche ladite bague ou rebord avec une pièce de forme conjuguée solidaire de la poche collectrice.

Dans d'autres dispositifs connus, la poche de recueil des déchets ou des fluides corporels est garnie d'un anneau ou analogue en un matériau adhésif sensible à la pression et ladite poche est mise en place en faisant adhérer l'anneau à un moyen protecteur fixé au corps de l'utilisateur, également sous l'action d'une pression de l'utilisateur.

Cependant, étant donné que la zone voisine de l'ouverture d'ostomie est sensible, généralement douloureuse, l'application d'une pression ne fait qu'accroître le malaise de l'utilisateur, de sorte que ni l'un ni l'autre type de dispositif mentionné ci-dessus n'est entièrement satisfaisant.

En outre, un montage avec pression insuffisante ne garantit pas l'étanchéité du dispositif, avec les conséquences extrêmement désagréables qui en résultent pour l'utilisateur.

Aussi a-t-on déjà proposé, par exemple dans GB-A-1 021 145, un dispositif dans lequel une poche de recueil est reliée à un disque de montage comportant un moyeu fileté par un anneau à visser, de sorte que l'on ne fait pas appel à la pression pour le montage amovible de la poche sur l'élément conjugué du dispositif fixé au corps de l'utilisateur. Dans ce dispositif, toutefois, l'étanchéité indispensable du dispositif est obtenue par coopération du moyeu et de l'anneau de sorte que si elle est suffisante le dispositif est nécessairement à tolérances serrées donc la poche difficile à mettre en place et à retirer. De plus, la poche n'est pas prévue solidaire de l'anneau, mais doit être rapportée sur celui-ci par l'utilisateur, ce qui n'est pas toujours sans inconvénients.

C'est, par conséquent, un but général de l'invention, de fournir un dispositif d'ostomie perfectionné qui soit d'utilisation plus simple et plus sûre que celle des dispositifs connus.

C'est, encore, un but de l'invention de fournir un tel dispositif qui trouve application non seulement dans des cas d'ostomie abdominale, mais également pour des ostomies du système urinaire dans lesquels on met parfois en oeuvre un système semi-permanent, c'est-à-dire dans lequel la poche ou sac de recueil des urines est muni d'un moyen d'évacuation de son contenu, de sorte que le dispositif n'est pas renouvelé à chaque miction, mais est conservé par l'utilisateur pendant un ou plusieurs jours.

Un dispositif d'ostomie selon l'invention comportant deux éléments dont l'un est prévu pour être fixé autour d'une ouverture artificielle du corps de l'utilisateur à l'aide d'un moyen adhésif sensible à la pression, d'une ceinture ou de toute autre manière analogue et dont l'autre comprend une poche collectrice de fluides et/ou de déchets corporels destinée à être accouplée de manière amovible audit premier élément à l'aide de moyens de vissage ménagés sur deux embouts dont un premier est fixé sur un patin formant un protecteur cutané de la zone péristomiale et autour d'une ouverture dudit patin, tandis que le second est fixé autour d'une ouverture de la poche collectrice est caractérisé en ce que ledit premier embout, -qui peut également être fixé autour du débouché d'une ou de sonde(s) ou analogue(s) qui en est(sont) solidaire(s)-, de même que ledit second embout sont munis de moyens destinés à assurer l'étanchéité du dispositif lors de la solidarisation des deux éléments entre eux par vissage, moyens qui comprennent une lèvre annulaire en saillie sur la face interne de l'un des embouts coaxiale à l'axe de rotation des moyens de vissage et raccordée, par une de ses faces au moins, sensiblement au voisinage de la moitié de la longueur de l'embout auquel elle est associée.

Dans une réalisation préférée, la lèvre annulaire à section droite triangulaire et inclinée sur ledit axe est propre à coopérer avec un manchon de dimensions conjuguées de celles de ladite lèvre ménagé sur l'autre embout.

Dans une autre réalisation préférée, également, ladite lèvre annulaire est prévue entre deux surfaces cylindriques de l'embout auquel elle est reliée.

Complémentairement, l'embout qui porte la lèvre est à surface conique depuis son orifice externe jusqu'à l'arête circulaire limitant l'extrèmité libre de ladite lèvre annulaire.

Pour faciliter l'utilisation du dispositif selon l'invention, on prévoit complémentairement que la longueur du manchon, mesurée parallèlement à son axe, soit différente de celle d'une jupe qui porte les filets des moyens de vissage.

Ces derniers sont choisis, selon une autre réalisation préférée, pour que les filets d'un embout présentent un léger jeu par rapport aux filets de l'autre embout, avec pour conséquence l'élimination de tout risque de grippage et une meilleure facilité d'emploi.

Dans une forme de réalisation particulièrement avantageuse, la disposition des filets de vis des deux embouts est également choisie de manière telle que la prise desdits filets n'ait lieu qu'après un léger angle de rotation à vide, ce qui contribue également au bon positionnement des deux embouts l'un par rapport à l'autre.

Selon une autre réalisation préférée, on prévoit que les filets d'un des embouts soient munis de discontinuités, comme des encoches, régulièrement décalées au point de vue angulaire et que l'autre embout présente des bossages de forme et dimensions conjuguées desdites discontinuités entre deux filets de vis.

On obtient ainsi un obstacle au blocage, d'une part et, d'autre part, un moyen de sûreté anti-retour dans la condition d'assemblage des embouts.

Pour encore faciliter le positionnement des deux embouts l'un par rapport à l'autre, dans une forme de réalisation avantageuse, il est prévu aussi de munir l'embout fileté solidaire de la poche de moyens en facilitant sa préhension, comme des rainurages ou moletages disposés sur la périphérie de l'embout ainsi que des saillies qui ménagent des moyens de repère, lesquels sont de préférence disposés dans le plan diamétral qui est celui de début des filets de vissage dudit embout.

Dans une telle réalisation, l'embout conjugué de celui présentant les moyens de repère mentionnés est lui aussi muni de moyens de repérage, lesquels sont disposés de manière telle que, après liaison des deux embouts entre eux, l'angle formé par le plan diamétral des moyens de repère du premier embout et le plan diamétral des moyens de repérage du second embout est un multiple de l'angle de rotation des deux embouts l'un par rapport à l'autre.

Dans une réalisation particulièrement préférée les moyens de vissage sont du type à "quart de tour", c'est-à-dire que l'accouplement des deux éléments entre eux est rendu opératoire par une rotation de 90°.

Une autre réalisation préféré prévoit aussi une poche de recueil de fluides et/ou déchets corporels notamment pour colostomie, iléostomie, ou urostomie propre à entrer dans la construction d'un dispositif tel que défini ci-dessus et caractérisée en ce qu'elle comprend, autour d'une ouverture qu'elle présente dans une de ses parois, un embout muni de moyens de filetage en saillie sur la face externe de ladite paroi et solidaire de celle-ci.

Finalement une autre réalisation vise également un protecteur cutané propre à entrer dans la constitution d'un tel dispositif d'ostomie et caractérisé en ce qu'il comprend, sur un patin propre à être fixé au corps de l'utilisateur à l'aide d'un moyen sensible à la pression, d'une ceinture ou de toute autre manière analogue, un embout portant des moyens de filetage, en saillie sur la face dudit patin opposée à celle destinée à venir au contact du corps de l'utilisateur avec, sur ledit embout, des une lérre élastignement déformable propre à assurer l'étanchéité du montage de la poche collectrice sur le protecteur lors du vissage des embouts l'un sur l'autre.

L'invention sera bien comprise par la description qui suit, faite à titre d'exemple et en référence au dessin annexé dans lequel :

- la figure 1 est une vue schématique, en perspective, des deux éléments constitutifs d'un dispositif selon l'invention pour une première forme de réalisation de celui-ci ;
- la figure 2 est une vue en plan de dessous d'un embout de dispositif selon l'invention ;
- la figure 3 est une vue en plan de l'autre embout ;
- la figure 4 est une vue partielle en développé des moyens de filetage de l'embout montré sur la figure 2 ;
- la figure 5 est une section suivant la ligne 5-5 de la figure 4, à plus grande échelle ;
- la figure 5A est une section suivant la ligne 5A-5A de la figure 4, à plus grande échelle ;
- la figure 6 est une vue analogue à celle de la figure 4 mais pour la pièce montrée sur la figure 3 ;
- la figure 6A est une section suivant la ligne 6A-6A de la figure 6, à plus grande échelle ;
- la figure 7 est une vue partielle, à plus grande échelle et en coupe, du dispositif montré sur la figure 1 ;
- la figure 8 est une vue schématique, en perspective, d'un des éléments d'une autre réalisation du dispositif selon l'invention ;
- la figure 9 est une vue en coupe d'un dispositif selon l'invention comprenant l'élément montré sur la figure 8.

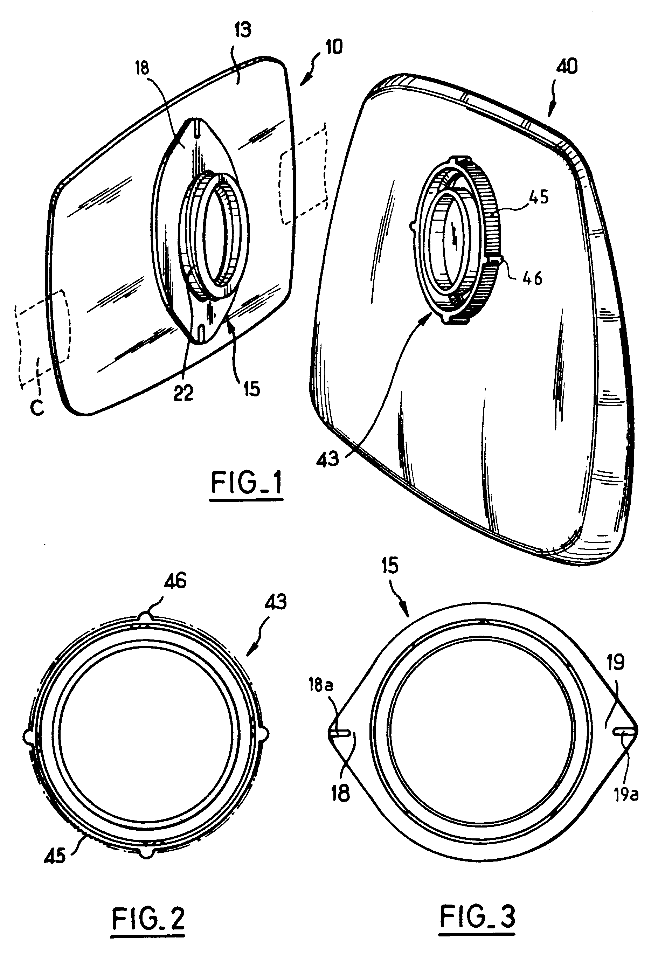

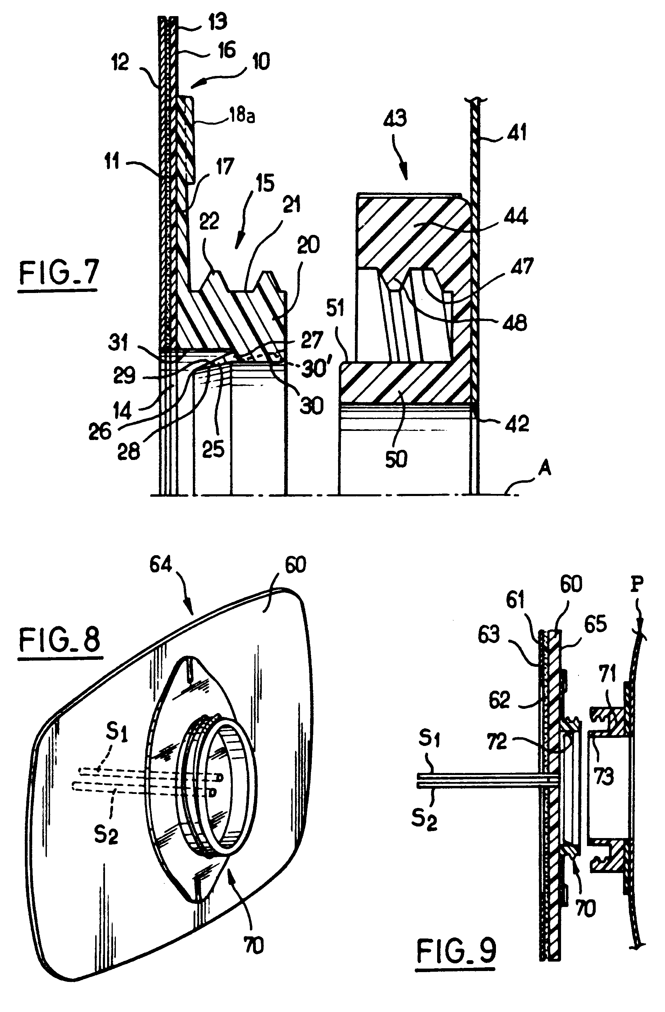

On se réfère d'abord aux figures 1 à 7 relatives à une première réalisation d'un dispositif d'ostomie perfectionné selon l'invention. Celui-ci comprend un élément 10, prévu pour être fixé autour d'une ouverture artificielle du corps de l'utilisateur par une ceinture C ou un moyen analogue ou, en variante, à l'aide d'une garniture 11 en un matériau adhésif sensible à la pression, en soi connue, et qui est généralement protégée, aussi longtemps que le dispositif n'est pas mis en oeuvre, par une pellicule 12 qui peut être aisément retirée par pelage, figure 7.

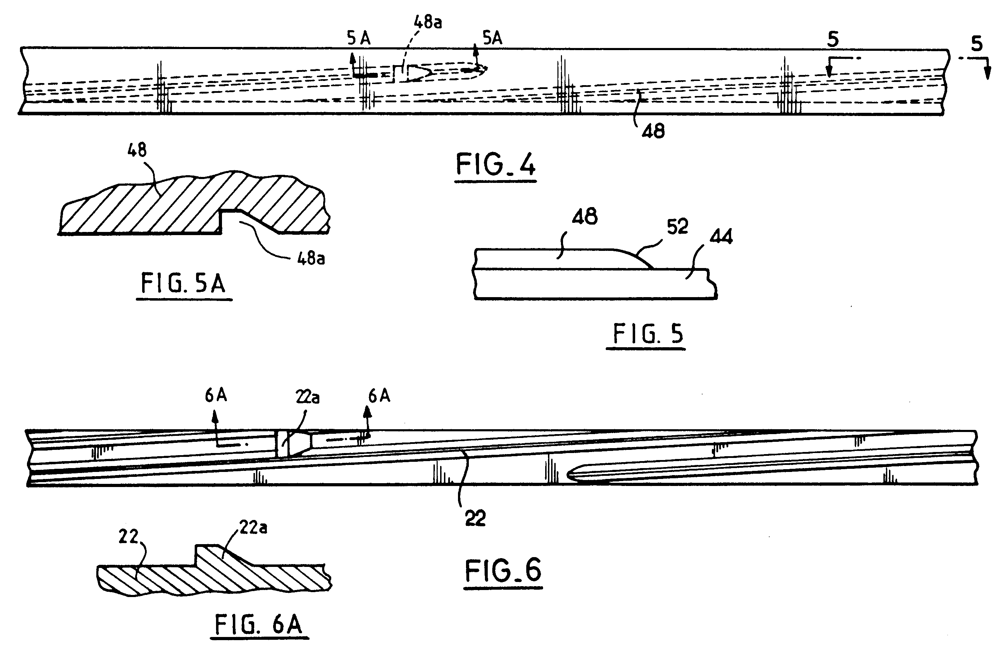

Quel que soit son mode d'assujettissement au corps de l'utilisateur, l'élément 10 comprend essentiellement une partie ou patin 13 en un matériau souple, par exemple de forme ronde, rectangulaire ou polygonale destiné à former un protecteur cutané de la zone péristomiale autour de laquelle il est placé et maintenu, avec son ouverture 14 concentrique ou sensiblement concentrique à la stomie, et un embout 15 solidaire du patin 13, en saillie sur la face 16 de ce patin opposée à celle portant le matériau adhésif 11 ou, lorsqu'un tel matériau n'est pas présent, opposée à celle en contact avec la peau de l'utilisateur. Dans la réalisation décrite et représentée l'embout 15 comprend une embase 17, de forme générale circulaire avec toutefois, en des zones diamétralement opposées, de légères protubérances 18 et 19 qui portent de légères surépaisseurs radiales, 18a et 19a, respectivement. L'embout 15 comprend aussi, venue d'une pièce avec ladite embase 17, par exemple lors d'une fabrication par moulage de matière plastique, une jupe 20 portant sur sa surface externe 21 des filets de vis 22, figures 6 et 6A et, sur sa surface interne, une lèvre 25 élastiquement déformable. Celle-ci est à section droite triangulaire, reliée au corps de la jupe 20 par une base 27 avec des faces 28 et 29 inclinées sur ledit axe et qui se coupent suivant un angle de faible valeur pour définir une arête 26, circulaire; le diamètre de cette dernière est légèrement inférieur à celui d'une première surface cylindrique 30, d'axe A, qui se raccord à la base 27 laquelle se raccorde ausi à une seconde surface cylindrique 31, d'axe A, et de diamètre légèrement supérieur à celui de la surface 30.

En variante, et comme montré en trait pointillé sur la figure 7, la face 28 de la lèvre se raccorde à une surface conique 30′ dont l'angle au sommet est au plus égal à celui définissant la face 28 et dans son prolongement.

Dans la réalisation illustrée d'un dispositif à "quart de tour", les filets de vis 22 discontinus de l'embout 15 sont au nombre de quatre, décalés de 90° les uns par rapport aux autres, quatre bossages 22a régulièrement disposés au point de vue angulaire étant prévus entre deux filets contigüs, figures 6 et 6A. L'embout est rendu solidaire du patin 13 par soudure, par exemple du type thermique ou haute fréquence ou encore par collage par l'intermédiaire de films compatibles avec le matériau constitutif de l'embout 15 et qui sont prévus sur la face 16 du patin.

Ce dernier peut être constitué à partir d'une masse adhésive hydrophile d'une épaisseur comprise entre 0,5 et 3 mm, ou à partir d'une masse adhésive acrylique très mince dont l'épaisseur est comprise entre quelques microns et 200 microns environ ou encore par combinaison de ces deux éléments, c'est-à-dire, en prévoyant autour de la stomie une gomme adhésive hydrophile et, à la périphérie de celle-ci, une masse adhésive acrylique mince.

L'embout 15 peut être réalisé par moulage de polyéthylène, haute ou basse densité, ou de copolymère d'éthylène et d'acétate de vinyle (EVA) ou de polychlorure de vinyle (PVC) ou d'un polyamide et les films compatibles pour la fixation de l'embout sur le protecteur cutané sont alors avantageusement des films de polyéthylène, de PVC, de polyamide, ou des films barrière complexes ou des nontissés à base de polyester, de polypropylène et/ou de polyéthylène.

Avec l'élément 10 du dispositif d'ostomie selon l'invention est propre à coopérer, de manière amovible, une poche 40, figure 1, de recueil de fluides et/ou déchets corporels évacués au travers de l'embout 15, et qui comprend une paroi 41, figure 7, percée d'un trou 42 par lequel les fluides ou déchets corporels pénètrent à l'intérieur de la poche, laquelle peut être du type à jeter, ou à vider, en fonction des desiderata de la pratique. Elle peut être réalisée en un film de polyéthylène, ou de PVC, ou de polyamide (comme celui connu sous la marque déposée RILSAN) ou en un film barrière complexe du type polyéthylène/EVA/polychlorure de vinylidène/EVA/polyéthylène, comme ceux connus sous la marque déposée SARANEX de la Société DOW CHEMICAL, ou un film complexe du type EVA/polychlorure de vinylidène et EVA, comme ceux connus sous la marque CRYOVAC de la Société GRACE et sous la marque déposée SARANEX de la Société DOW CHEMICAL, ou encore un film complexe du type EVA/EVOH/EVA, ou en caoutchouc ou analogue. Conformément à l'invention, à la poche 40 est associé un embout 43, figures 2, 4, 4A, 5 et 7, avantageusement réalisé en la même matière plastique que l'embout 15, par exemple en polyéthylène haute densité et solidarisé de manière étanche avec la poche 40, sur laquelle il est soudé ou collé, comme indiqué ci-dessus, de manière que son axe soit coaxial à celui de l'ouverture 42.

Dans la réalisation décrite et représentée l'embout 43 comporte une jupe externe 44, à contour plan de forme générale circulaire (figure 2) avec de légères saillies 46 régulièrement réparties à la périphérie de ladite jupe à 90° les unes des autres pour servir de repères, la surface externe de la jupe étant rainurée ou moletée entre lesdites saillies comme montré schématiquement en 45. La face interne 47 de la jupe 44 porte, venus de moulage, des filets de vis discontinus 48, conjugués des filets 22 de l'embout 15 et qui, dans l'exemple de réalisation sont au nombre de quatre, figure 4, régulièrement décalés de 90° les uns par rapport aux autres avec, sur lesdits filets, des encoches 48a, figure 5A, de forme et de dimensions conjuguées de celles des bossages 22a.

L'embout 43 comprend également, venu d'une pièce avec la jupe 44, un manchon 50, cylindrique, dont la longueur mesurée parallèlement à l'axe A est plus grande que celle de la jupe 44 et dont le diamètre de la face externe 51 est inférieur au diamètre de la partie 30 mais légèrement supérieur à celui de l'arête 26 de sorte que ledit manchon est propre à déformer élastiquement la lèvre 25 avec laquelle il coopère lorsque l'embout 43 est vissé sur l'embout 15 en vue de fixer à étanchéité la poche 40 sur l'élément 10 à protecteur cutané 13.

En réalisant les filets 48 de l'embout 43 de manière à ce qu'ils présentent un léger jeu par rapport aux filets 22 de l'embout 15, d'une part, en donnant à l'entrée de ces filets une section arrondie, comme montré en 52, figure 5, et en prévoyant également, d'autre part, une disposition des filets telle que leur venue en prise n'a lieu qu'après un léger angle de rotation à vide, on élimine tout risque de grippage tant à la mise en place qu'au retrait de la poche de recueil 40, ce qui rend le dispostif d'une grande facilité d'utilisation aussi bien dans le cas de colostomie, d'iléostomie, que d'urostomie. A la facilité d'utilisation du dispositif contribue la présence, sur l'un et l'autre des embouts 15 et 43, de repères ménagés par et sur les protubérances 18 et 19 de l'embout 15 et les saillies 46 de l'embout 43 également muni du rainurage ou moletage 45 facilitant sa préhension. Les encoches 48a et les bossages 22a s'opposent avantageusement au blocage des deux embouts l'un par rapport à l'autre tout en ménageant des crans de sûreté anti-retour qui font obstacle à une désolidarisation intempestive en cours d'utilisation du dispositif.

On se réfère maintenant aux figures 8 et 9 relatives à une autre réalisation d'un dispositif selon l'invention, plus particulièrement prévue pour le recueil d'urine, par exemple de personnes urétérostomisées. Le dispositif 62 comprend un protecteur cutané 60 dont une face 61 porte une garniture en un matériau adhésif sensible à la pression, avantageusement protégé par une pellicule 63 aussi longtemps que le dispositif n'est pas mis en oeuvre et, solidaire(s) de ce protecteur 60, une ou deux sondes $S_1$ et $S_2$ destinée(s) à être introduite(s) dans une stomie d'uretère pour le traitement d'urostomie simple ou double.

Selon l'invention, l'élément 64 comportant le protecteur cutané 60 et la ou les sondes S présente en saillie sur sa face 65, - opposée à la facé 61 -, un embout 70 montré schématiquement sur les figures 8 et 9 et dont la structure est semblable, sinon identique, à celle de l'embout 15 de la réalisation précédente, ledit embout étant prévu pour le montage, à étanchéité, d'une poche de recueil P, figure 9, munie d'un embout 71 de stucture analogue ou identique à celle de l'embout 43 de la réalisation précédente. Dans cette réalisation, également, c'est par l'intermédiaire d'une lèvre 72 ménagée sur la face interne de l'embout tubulaire 70 et avec laquelle est propre à coopérer un manchon 73 de forme et dimensions conjuguées de l'embout 71 qu'est réalisée l'étanchéité.

L'embout 70 est fixé sur le protecteur cutané 60 par soudure, thermique ou à haute fréquence, ou par collage, comme indiqué ci-dessus, avantageusement avec utilisation de films compatibles entre l'embout et le protecteur cutané et l'embout 71 est fixé sur la poche P par un procédé analogue de soudure ou collage.

De bons résultats ont été obtenus à l'aide d'un dispositif selon la figure 1 comportant une poche de recueil en SARANEX (une marque déposée de DOW CHEMICAL) et réalisé par moulage de polyéthylène haute densité dans lequel l'embout 15 avait un diamètre externe d'environ 75 mm, une longueur mesurée parallèlement à l'axe A d'environ 5 mm, un diamètre de la partie cylindrique 30 de 54,5 mm, un diamètre de la partie 31 de 56 mm et un diamètre de la lévre 26 de 53,5 mm, alors que le diamètre externe du manchon 50 était de 54 mm et son diamètre interne de 50 mm, le diamètre externe de l'embout 43 étant d'environ 70 mm pour quatre filets à droite d'un pas de dix.

**Revendications**

1. Dispositif d'ostomie comportant deux éléments (10, 40) dont l'un (10) est prévu pour être fixé autour d'une ouverture artificielle du corps de l'utilisateur à l'aide d'un moyen adhésif sensible à la pression, d'une ceinture (C) ou de toute autre manière analogue et dont l'autre (40) comprend une poche collectrice de fluides et/ou de déchets corporels destinée à être accouplée de manière amovible audit premier élément (10) à l'aide de moyens de vissage ménagés sur deux embouts (15, 70 ; 43, 71) dont un premier (15, 70) est fixé sur un patin (13, 60) formant un protecteur cutané de la zone péristomiale et autour d'une ouverture dudit patin (13, 60), tandis que le second (43, 71) est fixé autour d'une ouverture de la poche collectrice (40, P), caractérisé en ce que ledit premier embout (15, 70), -qui peut également être fixé autour du débouché d'une ou de sonde(s) ou analogue(s) qui en est(sont) solidaire(s)-, de même que ledit second embout (43, 71) sont munis de moyens (25, 72 ; 50, 73) destinés à assurer l'étanchéité du dispositif lors de la solidarisation des deux éléments entre eux par vissage, moyens qui comprennent une lèvre annulaire en saillie sur la face interne (30, 31) de l'un des embouts (15, 70), coaxiale à l'axe (A) de rotation des moyens de vissage et raccordée, par une de ses faces au moins, sensiblement au voisinage de la moitié de la longueur de l'embout (15, 70) auquel elle est associée.

2. Dispositif selon la revendication 1, caractérisé en ce que la lèvre annulaire à section droite triangulaire (25, 72) et inclinée sur ledit axe (A) est propre à coopérer avec un manchon (50, 73) de dimensions conjuguées de celles de ladite lèvre ménagé sur l'autre embout (71, 43).

3. Dispositif selon la revendication 2, caractérisé en ce que ladite lèvre annulaire (25, 72) est prévue entre deux surfaces cylindriques (30, 31) de l'embout auquel elle est reliée.

4. Dispositif selon la revendication 2, caractérisé en ce que ledit embout (15, 70) qui porte la lèvre (25, 72) est à surface conique depuis son orifice externe jusqu'à l'arête circulaire limitant l'extrémité libre de ladite lèvre annulaire (25, 72).

5. Dispositif selon la revendication 2, caractérisé en ce que la longueur du manchon (50, 73) mesurée parallèlement à son axe (A), est différente de celle d'une jupe (44) qui porte les filets (48) des moyens de vissage de l'embout (43, 71) dont est solidaire le manchon 50, 73).

6. Dispositif selon l'une quelconque des revendications précédentes, caractérisé en ce que les filets (22) d'un embout (15) présentent un léger jeu par rapport aux filets (48) de l'autre embout (43) pour éliminer les risques de grippage lors de la solidarisation et/ou de l'enlèvement d'un embout par rapport à l'autre.

7. Dispositif selon l'une quelconque des revendications précédentes, caractérisé en ce que les filets de vis (22, 48) des deux embouts (15, 43 ; 70, 71) sont choisis de manière telle que la prise de filets n'ait lieu qu'après un léger angle de rotation à vide.

8. Dispositif selon l'une quelconque des revendications précédentes, caractérisé en ce que pour s'opposer au blocage des deux embouts après assemblage et pour procurer une sûreté anti-retour en cette condition, on prévoit des encoches (48a) sur les filets (48) d'un des embouts (43) et des bossages de forme et dimensions conjuguées (22a) entre les filets (22) de l'autre embout (15).

9. Dispositif selon l'une quelconque des revendications précédentes, caractérisé en ce que l'embout fileté (43, 71) solidaire de la poche (40, P) de recueil de fluides et/ou des déchets corporels présente des moyens en facilitant sa préhension, comme un rainurage ou moletage (45) disposé sur la périphérie dudit embout (43), ainsi que des saillies (46) qui ménagent des moyens de repère diamétralement opposés et de préférence disposés dans le plan diamétral qui est celui du début des filets des moyens de vissage dudit embout (43).

10. Dispositif selon l'une quelconque des revendications précédentes, caractérisé en ce que les moyens de vissage sont du type à "quart de tour" c'est-à-dire que l'accouplement des deux éléments entre eux est rendu opératoire par une rotation de 90°.

11. Poche de recueil de fluides et/ou de déchets corporels notamment pour colostomie, iléostomie, ou urostomie, propre à entrer dans la constitution d'un dispositif selon l'une quelconque des revendications précédentes, caractérisée en ce qu'elle comprend autour d'une ouverture (42) qu'elle présente dans une de ses parois (41) un embout (43, 71) muni de moyens de filetage (48) et de moyens (50, 73) formant étanchéité, ledit embout faisant saillie sur la face externe de ladite paroi dont il est solidaire de fabrication.

12. Dispositif de protecteur cutané propre à entrer dans la constitution d'un dispositif d'ostomie selon l'une quelconque des revendications 1 à 10, caractérisé en ce qu'il comprend, sur un patin (13, 60) destiné à être fixé au corps de l'utilisateur à l'aide d'un adhésif sensible à la pression (11, 62) une ceinture (C) ou de toute autre manière analogue un embout (15, 70) portant des moyens de filetage (22) en saillie sur la face dudit patin opposée à celle destinée à venir au contact du corps de l'utilisateur et, sur ledit embout, une lèvre élastiquement déformable (25, 72) propre à assurer l'étanchéité du montage de la poche collectrice (40, -P) sur ledit protecteur lors du vissage des embouts (15, 70 ; 43, 71) l'un sur l'autre.

**13.** Dispositif selon la revendication 12, caractérisé en ce qu'il comporte une ou des sonde(s) (S) propre(s) à être introduite(s) dans une ou deux stomie(s) d'uretère(s) et solidaire(s) du patin (60) du protecteur cutané et/ou de l'embout (70) dudit protecteur.

**Claims**

**1.** An ostomy device comprising two elements (10, 40), one of which (10) is designed to be fixed around the artificial opening of the body of the user by means of a belt (C), pressure sensitive adhesive tape, or any other analogous means, and the other of which (40) includes a collector bag of collecting body fluids and/or wastes and designed to be removeably coupled to said first element (10) using screw-fastening means provided on two endpieces (15, 70; 43, 71), with a first endpiece (15, 70) being fixed on a plate (13, 60) and around an opening through said plate (13, 60), which plate constitutes a skin protector for the peristomial zone, while the second endpiece (43, 71) is fixed around an opening of the collector bag (40, P), the device being characterized in that both said first endpiece (15, 70) - which may also be fixed around the outlet of one or more probes or the like which are fixed thereto - and said second endpiece (43, 71) are provided with means (25, 72; 50, 73) designed to provide sealing of the device when the two elements are fixed together by screw fastening, which means comprise an annular lip coaxial with the axis of rotation (A) of the screw-fastening means, projecting from the inside face (30, 31) of one of the endpieces (15, 70), and connected by at least one of its faces substantially in the vicinity of halfway along the endpiece (15, 70) with which it is associated.

**2.** A device according to claim 1, characterized in that said annular lip of triangular right cross-section (25, 72) and inclined relative to said axis (A) is suitable for co-operating with a sleeve (50, 73) provided on the other endpiece (71, 43) and of dimensions which are complementary to those of said lip.

**3.** A device according to claim 2, characterized in that said annular lip (25, 72) is provided between two cylindrical surfaces (30, 31) of the endpiece to which it is connected.

**4.** A device according to claim 2, characterized in that said endpiece (15, 70) which carries the lip (25, 72) has a surface which is conical from its outer orifice to the circular edge delimiting the free end of said annular lip (25, 72).

**5.** A device according to claim 2, characterized in that the length of the sleeve (50, 73) measured parallel to its axis (A) is different from the length of a skirt (44) carried by the threads (48) of the screw-fastening means of the endpiece (43, 71) to which the sleeve (50, 73) is fixed.

**6.** A device according to any preceding claim, characterized in that the threads (22) of one of the endpieces (15) leave a small degree of play relative to the threads (48) of the other endpiece (43) in order to eliminate the risk of seizing when one of the endpieces is being fitted to and/or removed from the other.

**7.** A device according to any preceding claim, characterized in that the screw threads (22, 48) of the two endpieces (15, 43; 70, 71) are selected in such a manner that the threads engage only after rotation through a small dead angle.

**8.** A device according to any preceding claim, characterized in that in order to prevent the two endpieces from jamming after being assembled while providing non-return security in this condition, notches (48a) are provided on the threads (48) of one of the endpieces (43) and projections (22a) of complementary shape and size are provided between the threads (22) of the other endpiece (15).

**9.** A device according to any preceding claim, characterized in that the threaded endpiece (43, 71) fixed to the collector bag (40, P) for collecting body fluids and/or wastes presents means for facilitating grasping thereof, e.g. grooving or knurling (45) disposed on the periphery of said endpiece (43), together with projections (46) providing diametrically opposite reference means preferably disposed in the diametrical plane corresponding to the starts of the threads of the screw fastening means of said endpiece (43).

**10.** A device according to any preceding claim, characterized in that the screw-fastening means are of the "quarter-turn" type, i.e. the two elements are operationally coupled to each other after rotation through 90°.

**11.** A collector bag for collecting body fluids and/or wastes, in particular for colostomy, ileostomy, or urostomy, and suitable for use in a device according to any preceding claim, the bag being characterized in that it comprises an endpiece (43, 71) extending around an opening (42) provided in one of the walls (41) of the bag, the endpiece being provided with thread means (48) and with sealing means (50, 73), said endpiece projecting from the outside face of said wall to which it is fixed on manufacture.

12. A skin protector device suitable for forming a part of an ostomy device according to any one of claims 1 to 10, the skin protector device being characterized in that it comprises an endpiece (15, 70) on a plate (13, 60) for fixing to the body of the user by a belt (C), by pressure sensitive adhesive (11, 62), or by any other analogous means, said endpiece (15, 70) bearing thread means (22), and projecting from the face of said plate which is opposite to the face intended to come into contact with the body of the user, said endpiece further carrying a resiliently deformable lip (25, 72) suitable for providing sealing in the assembly of the collector bag (14, P) on said skin protector device when the endpieces (15, 70; 43, 71) are screwed together.

13. A device according to claim 12, characterized in that it includes one or more probes (S) suitable for insertion into one or two ureterostomies and fixed to the plate (60) of the skin protector and/or to the endpiece (70) of said protector.

**Patentansprüche**

1. Vorrichtung für einen künstlichen Körperausgang, welche aus zwei Elementen (10, 40) besteht, wovon eines (10) rund um einen künstlichen Ausgang des Körpers des Verwenders mit Hilfe eines druckempfindlichen anhaftenden Mittels, eines Gürtels (C) oder jedes anderen Mittels befestigbar ist und das andere (40) einen Sammelbeutel für Flüssigkeiten und/oder körperliche Ausscheidungen aufweist, welcher an das erste Element (10) lösbar mittels Schraubvorrichtungen anschließbar ist, die auf zwei Ansatzstücken (15, 70; 43, 71) angebracht sind, wovon das erste (15, 70) rund um eine Öffnung einer Unterlage (13, 60) angeordnet ist, die einen Hautschutz der peristomialen Zone bildet, während das zweite (43, 71) rund um eine Öffnung des Semmelbeutels (40, P) angeordnet ist, dadurch gekennzeichnet, daß sowohl dieses erste Ansatzstück (15, 70), welches auch rund um den Ausgang einer oder mehrerer Sonden oder ähnliches, die damit fest verbunden sind, befestigt werden kann, als auch dieses zweite Ansatzstück (43, 71) Mittel (25, 72; 50, 73) aufweisen, welche die Abdichtheit der Vorrichtung bei Verschraubung der beiden Elemente gewährleisten, Mittel, die aus einer ringförmigen Lippe bestehen, die auf der Innenseite (30 - 31) eines dieser Ansatzstücke (15, 70) vorspringt und koaxial zur Drehachse (A) der Verschraubvorrichtungen verläuft und mit einer ihrer Flächen zumindest ungefähr auf der Höhe der Hälfte der Länge des Ansatzstückes (15, 70) verbunden ist, dem sie zugeordnet ist.

2. Vorrichtung nach Anspruch 1, dadurch gekennzeichnet, daß die ringförmige Lippe, die einen dreieckigen Querschnitt (25, 72) aufweist und schräg zu der Achse (A) verläuft, mit einer Muffe (50, 73) mit Abmessungen zusammenwirken kann, die auf jene der auf dem anderen Ansatzstück (71, 43) angebrachten Lippe abgestimmt sind.

3. Vorrichtung nach Anspruch 2, dadurch gekennzeichnet, daß diese ringförmige Lippe (25, 72) zwischen zwei Zylinderflächen (30, 31) des Ansatzstückes, mit welchem sie verbunden ist, vorgesehen ist.

4. Vorrichtung nach Anspruch 2, dadurch gekennzeichnet, daß das Ansatzstück (15, 70), welches die Lippe (25, 72) aufweist, zwischen seiner äußeren Öffnung und der kreisförmigen Berandung, die das freie Ende dieser ringförmigen Lippe (25, 72) abgrenzt, eine Kegelfläche ist.

5. Vorrichtung nach Anspruch 2, dadurch gekennzeichnet, daß die Länge der Muffe (50, 73), längs ihrer Achse (A) gemessen, ungleich derjenigen einer Hülse (44) ist, welche die Gewinde (48) der Verschraubvorrichtungen des Ansatzstückes (43, 71) trägt, mit welchem die Muffe (50, 73) fest verbunden ist.

6. Vorrichtung nach einem der vorgehenden Ansprüche, dadurch gekennzeichnet, daß die Gewinde (22) eines Ansatzstückes (15) ein leichtes Spiel gegenüber den Gewinden (48) des anderen Ansatzstückes (43) aufweisen, um das Risiko des Festfressens während des Verschraubens und/oder des Lösens eines Ansatzstückes vom anderen zu vermeiden.

7. Vorrichtung nach einem der vorgehenden Ansprüche, dadurch gekennzeichnet, daß die Schraubgewinde (22, 48) der beiden Ansatzstücke (15, 43; 70, 71) derart gewählt sind, daß das Greifen der Gewinde erst nach Drehung um einen kleinen Leerlaufwinkel erfolgt.

8. Vorrichtung nach einem der vorgehenden Ansprüche, dadurch gekennzeichnet, daß auf den Gewinden (48) eines der Ansatzstücke (43) Vertiefungen (48a) und in den Gewinden (22) des anderen Ansatzstückes (15) Nocken von abgestimmter Form und Abmessung (22a) vorgesehen sind, um die Blockierung der beiden Ansatzstücke nach Verschraubung zu vermeiden und, um eine Rückdrehsicherung in dieser Lage zu geben.

9. Vorrichtung nach einem der vorgehenden Ansprüche, dadurch gekennzeichnet, daß das schraubbare Ansatzstück (43, 71), welches fest mit dem Sammelbeutel (40, P) für Flüssigkeiten und/oder körperliche Ausscheidungen verbunden ist, Vorrichtungen aufweist, die sein Greifen erleichtern, wie eine Riffelung oder Rändelung (45), die auf dem Umfang dieses Ansatzstückes (43) angeordnet ist, sowie Vorsprünge (46), welche diametral gegenüberliegenden Markierungen bilden und welche vorzugsweise in jener Diametralebene angeordnet sind, die den Steigungsbeginn der Gewinde der Verschraubvorrichtungen dieses Ansatzstückes (43) einschließt.

10. Vorrichtung nach einem der vorgehenden Ansprüche, dadurch gekennzeichnet, daß die Verschraubvorrichtungen der Gruppe der "Vierteldrehung" angehören, d.h. daß das Verbinden der beiden Elemente miteinander mittels einer 90°-Drehung erfolgen kann.

11. Sammelbeutel für Flüssigkeiten und/oder körperliche Ausscheidungen, insbesondere für Kolostomie, Ileostomie, oder Urethrostomie, der für den Aufbau einer Vorrichtung nach einem der vorgehenden Ansprüche geeignet ist, dadurch gekennzeichnet, daß er rund um eine Öffnung (42), die er in einer seiner Wände (41) aufweist, ein Ansatzstück (43, 71) besitzt, welches schraubbare Vorrichtungen (48) und Dichtungsmittel (50, 73) aufweist, wobei dieses Ansatzstück auf der äußeren Fläche dieser Wand, mit welcher er fertigungsmäßig fest verbunden ist, vorspringt.

12. Vorrichtung zum Hautschutz, welche für eine Vorrichtung für einen künstlichen Körperausgang nach einem der Ansprüche 1 bis 10 geeignet ist, dadurch gekennzeichnet, daß sie auf einer Unterlage (13, 60), welche an dem Körper des Vervenders mit Hilfe eines druckempfindlichen anhaftenden Mittels (11, 62), eines Gürtels (C), oder eines anderen ähnlichen Mittels befestigbar ist, ein Ansatzstück (15, 70) mit schraubbaren Vorrichtungen (22) aufweist, welches auf jener Fläche der Unterlage vorspringt, die gegenüber derjenigen liegt, die mit dem Körper des Verwenders in Kontakt stehen soll, und auf diesem Ansatzstück eine elastisch verformbare Lippe (25, 72) aufweist, die die Dichtheit des Anschlusses des Sammelbeutels (40, P) an diesen Schutz bei der Verschraubung beider Ansatzstücke (15, 70; 43, 71) gewährleistet.

13. Vorrichtung nach Anspruch 12, dadurch gekennzeichnet, daß sie eine oder mehrere Sonden (S) aufweist, welche in ein oder zwei Harnröhrenfisteln einführbar sind und welche mit der Unterlage (60) des Hautschutzes und/oder dem Ansatzstück (70) dieses Hautschutzes fest verbunden sind.

FIG_1

FIG_2

FIG_3

FIG. 4

FIG. 5A

FIG. 5

FIG. 6

FIG. 6A

EP 0 270 400 B1

FIG.7

FIG.8

FIG.9